# EUROPÄISCHE PATENTSCHRIFT

(11) **EP 2 223 709 B1**
(45) Veröffentlichungstag und Bekanntmachung des Hinweises auf die Patenterteilung: **02.12.2015**
(21) Anmeldenummer: 10001081.8
(22) Anmeldetag: 03.02.2010
(51) Int. Cl.: A61M 1/00

(54) **Saugkopf zur Absaugung von Blut**
Suction head for extracting blood
Tête aspirante destinée à aspirer le sang

(30) Priorität: 27.02.2009 DE 102009010941
(43) Veröffentlichungstag der Anmeldung: 01.09.2010
(73) Patentinhaber: BOIDA Kunststofftechnik GmbH, 35428 Langgöns (DE)
(72) Erfinder: Maier, Stefan, 35428 Langgöns (DE)
(74) Vertreter: Hoppe, Lars

(56) Entgegenhaltungen:
- US-A- 3 191 600
- US-A1- 2001 047 152
- US-A1- 2007 203 449

## Beschreibung

Die Erfindung bezieht sich auf einen Saugkopf zur Absaugung des während eines chirurgischen Eingriffs sich im Eingriffsbereich ansammelnden Blutes, insbesondere im Herz- und Thoraxbereich bei Herzoperationen, mit mehreren umfangsseitigen Längsschlitzen gemäß dem Oberbegriff des Anspruchs 1.

Während eines chirurgischen Eingriffs muss das sich im Operationsbereich, speziell bei einer Herzoperation im Thoraxbereich, ansammelnde Blut entfernt werden, wozu ein handelsüblicher Sauger mit einem Saugkopf in einer Einweg- oder Mehrwegausführung über einen Schlauch mit einer Saugvorrichtung verbunden ist.

Aus der Praxis ist ein mehrfach verwendbarer Saugkopf zur Absaugung des während eines chirurgischen Eingriffs sich im Eingriffsbereich ansammelnden Blutes mit mehreren umfangsseitigen Längsschlitzen bekannt, der aus einem rostfreien Stahl besteht und auf ein ebenfalls aus einem rostfreien Stahl bestehendes Saugrohr aufgeschraubt ist. Das Saugrohr wird über einen Saugschlauch mit einer Saugeinrichtung verbunden. Das Saugrohr ragt in den Saugkopf hinein, so dass zwischen der Außenwandung des Saugrohrs und der Innenwandung des Saugkopfes ein ringförmiger Strömungskanal ausgebildet ist und das abgesaugte Blut durch diesen Strömungskanal angesaugt und am freien Ende in das Saugrohr eingesaugt wird. Als problematisch erweist sich bei diesem Saugkopf die aufwändige Reinigung und die verhältnismäßig teure Herstellung insbesondere der Längsschlitze.

Im Weiteren ist ein einmal verwendbarer Saugkopf aus einem Kunststoff bekannt, der lediglich im Bereich seines freien Endes mit umfangsseitigen Längsschlitzen von verhältnismäßig geringer Länge versehen ist. Dieser Saugkopf weist aufgrund der Gestaltung der Längsschlitze ein unzureichendes Ergebnis auf.

Aus der US 2001/0047152 A1 ist ein Saugkopf der eingangs erwähnten Art bekannt, bei dem die Rippen des Innenteils dichtend in die Längsschlitze der Außenhülse eingreifen und als Absperr- und Saäuberungsorgan wirken. Diese Anordnung ermöglicht kein Absaugen von Fluiden.

Es ist Aufgabe der Erfindung, einen Saugkopf der eingangs genannten Art zu schaffen, der bei einer ausreichenden Saugleistung kostengünstig zu fertigen und einfach zu reinigen ist.

Erfindungsgemäß wird die Aufgabe durch die kennzeichnenden Merkmale des Anspruchs 1 gelöst.

Die Außenhülse lässt sich mit verhältnismäßig großen und leicht zu reinigenden Schlitzen kostengünstig fertigen und durch die Rippen des im Querschnitt sternförmigen Innenteils werden die Querschnitte der Längsschlitze derart reduziert, dass kein Gewebe angesaugt wird. Im Weiteren sind mehrere Strömungskanäle geschaffen, die den Aufbau eines unerwünscht hohen Unterdrucks verhindern. Darüber hinaus ist durch die Gestaltung der Querschnitte verhindert, dass das Blut beim Absaugen in Turbulenz gerät, wodurch rote Blutkörperchen beschädigt werden können.

Damit das abgesaugte Blut möglichst nicht geschädigt wird und beispielsweise zur Re-Infusion wieder verwendbar ist, ist vorzugsweise der frei durchströmbare Querschnitt zwischen den Längsschlitzen und den Rippen derart bemessen, dass rote Blutkörperchen beim Durchströmen nicht geschert werden. Die roten Blutkörperchen mit einem Durchmesser von ca. 5-8 µm durchströmen den Saugkopf ohne Scherung, d.h. sie unterliegen keiner traumatischen Beeinflussung. Somit kann das abgesaugte Blut nach einer entsprechenden Filtrierung in den Blutkreislauf zurückgeführt werden.

Erfindungsgemäß ist das rohrförmige Innenteil stirnseitig mit einem Saugrohr verbunden und in diesem endseitigen Verbindungsbereich fest mit der Außenhülse gekoppelt. Das Saugrohr erleichtert die Handhabung des Saugkopfes und ermöglicht dessen Anschluss an eine Saugpumpe oder dergleichen. Durch die feste Verbindung des Innenteils mit der Außenhülse ist ein unbeabsichtigtes Lösen dieser beiden Teile verhindert.

Um verschiedene Saugrohre bzw. Saugrohre unterschiedlicher Hersteller mit dem Saugkopf koppeln zu können, steht vorzugsweise das Innenteil kraftschlüssig mit dem Saugrohr in Verbindung, insbesondere über einen Luer-Lock-Anschluss. Bei der kraftschlüssigen Verbindung ist lediglich zu beachten, dass das Saugrohr fest mit dem Innenteil verbunden wird, damit sich der Saugkopf nicht unbeabsichtigt löst. Mit einem genormten Luer-Lock-Anschluss zwischen dem Saugkopf und dem Saugrohr ist ein großes Maß an Sicherheit gegen ein unabsichtliches Lösen der Verbindung sowie an Kompatibilität der Anschüsse verschiedener Hersteller gegeben.

Zur Schaffung eines relativ großen Bereichs, der sich zur Absaugung eignet, erstrecken sich die Rippen und die Längsschlitze, ausgehend von dem Verbindungsbereich, bis zu einer Spitze der Außenhülse. Erfindungsgemäß ist außerhalb des Verbindungsbereichs das Innenteil zu der Außenhülse bereichsweise beabstandet, um Strömungskanäle auszubilden. Demnach wird das Blut durch die Längsschlitze angesaugt, fließt in dem Ringraum zwischen dem Innenteil und der AuBenhülse zum freien Ende des Saugkopfes und wird durch das rohrförmige Innenteil abgeleitet.

Zur Vermeidung von Verletzungen weist die Spitze der Außenhülse einen Kugelradius auf. Die Außenhülse selbst bzw. der gesamte Saugkopf kann sich konisch zur Spitze verjüngend gestaltet sein, damit der Saugkopf auch bei beengten Platzverhältnissen einsetzbar ist.

In Ausgestaltung ist die Außenhülse an ihrer freien Stirnseite mit einer Saugöffnung versehen, die einen Querschnitt aufweist, der derart bemessen ist, dass Gase und Flüssigkeiten mit einer geringeren Viskosität als Blut eingesaugt werden. Durch diese stirnseitige Saugöffnung ist also zum einen vermieden, dass ein unerwünscht hoher Unterdruck entstehen kann, da beispielsweise Luft in den Saugkopf gelangen kann, und zum anderen können Flüssigkeiten abgesaugt werden, ohne dass Blut an dieser Stelle aus dem Saugkopf austreten kann.

Zur kostengünstigen Fertigung in großen Stückzahlen sind die Außenhülse und/oder das Innenteil aus Kunststoff gefertigt. Damit sowohl eine optische Kontrolle sowie eine einfache Handhabung bei der Absaugung als auch eine leichte Entformung bei der Fertigung möglich ist, ist bevorzugt die Außenhülse aus einem transparenten Kunststoff gefertigt und verjüngt sich zu ihrem freien Ende hin konisch. Durch die kostengünstige Herstellung des Saugkopfes aus Kunststoff ist seine Reinigung und Mehrfachverwendung nicht erforderlich. Vielmehr wird ein Wegwerfartikel bereitgestellt. Um jegliche Montage zu vermeiden, ist die Außenhülse gemeinsam mit dem Innenteil in einem Zwei-Komponenten-Spritzgußverfahren gefertigt.

Bevorzugt ist das Saugrohr aus einem Kunststoff gefertigt. Demnach kann auch das kostengünstige Saugrohr nach seiner einmaligen Verwendung entsorgt werden. Zur Verbesserung der Handhabbarkeit weist das Saugrohr ein Griffstück, insbesondere aus Kunststoff, auf. Selbstverständlich kann das Griffstück ergonomisch geformt sein.

Damit das Saugrohr einfach an bestehende System anschließbar ist, weist nach einer Weiterbildung das Saugrohr einen Luer-Konus, insbesondere mit Überwurfmutter für einen Luer-Lock-Anschluss, zur Kopplung mit einem Saugschlauch einer Saugvorrichtung auf.

Es versteht sich, dass die vorstehend genannten und nachstehend noch zu erläuternden Merkmale nicht nur in der jeweils angegebenen Kombination, sondern auch in anderen Kombinationen verwendbar sind. Der Rahmen der Erfindung ist nur durch die Ansprüche definiert.

Die Erfindung wird im Folgenden anhand eines Ausführungsbeispieles unter Bezugnahme auf die zugehörigen Zeichnungen näher erläutert. Es zeigt:

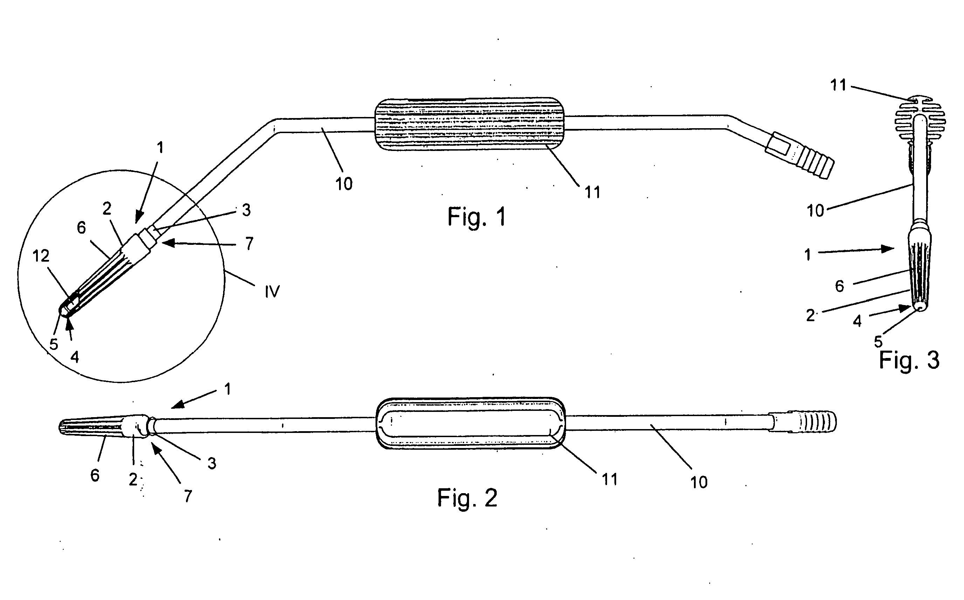

- Fig.1: eine Vorderansicht eines Saugrohrs mit einem erfindungsgemäßen Saugkopf,
- Fig.2: eine Draufsicht der Darstellung nach Fig. 1,
- Fig.3: eine Seitenansicht der Darstellung nach Fig. 1,

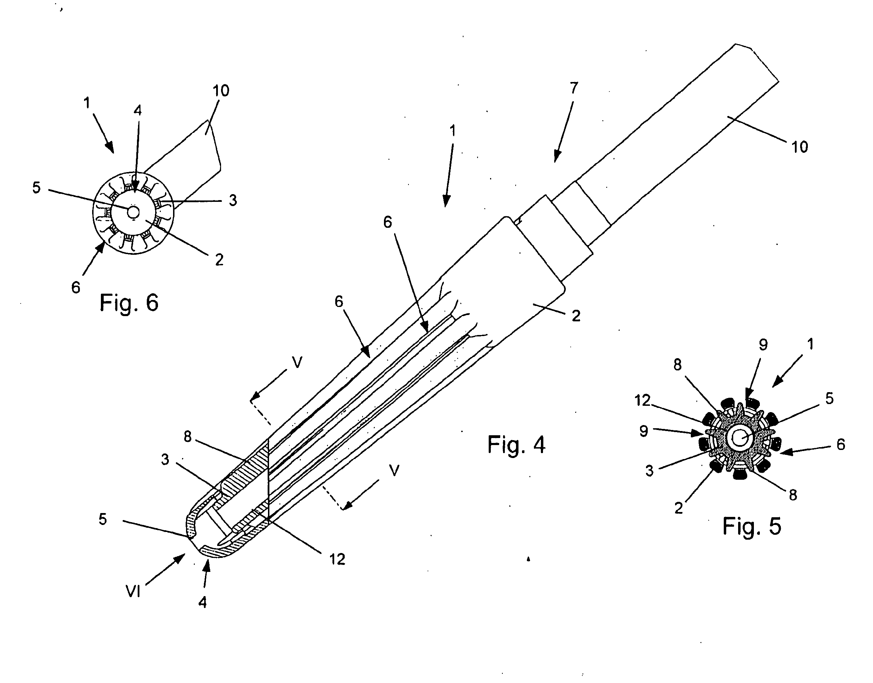

- Fig.4: eine vergrößerte Darstellung der Einzelheit IV nach Fig. 1,
- Fig.5: eine Schnittdarstellung gemäß der Linie V-V nach Fig. 5 und
- Fig.6: eine Ansicht in Richtung des Pfeils VI nach Fig.4.

Der Saugkopf 1 dient zur Absaugung des während eines chirurgischen Eingriffs sich im Eingriffsbereich ansammelnden Blutes und umfasst eine Außenhülse 2 mit einem eingesetzten rohrförmigen Innenteil 3. Die Spitze der Außenhülse 2 weist einen Kugelradius auf 4 und ist mit einer kreisförmigen Saugöffnung 5 versehen, deren Querschnitt derart bemessen ist, dass Gase und Flüssigkeiten mit einer geringeren Viskosität als Blut eingesaugt werden und demzufolge Blut nicht durch die Sauföffnung 5 aus dem Saugkopf 1 austreten kann. In die konische Außenhülse 2 sind gleichmäßig über den Umfang verteilte Längsschlitze 6 eingelassen, die sich von dem Kugelradius 4 über einen Großteil der Länge der Außenhülse 2, im Wesentlichen bis zu einem endseitigen Verbindungsbereich 7 mit dem Innenteil 3, erstrecken. In die Längsschlitze 6 erstrecken sich die Rippen 8 des im Querschnitt sternförmigen Innenteils 3, wobei sich Rippen 8 querschnittsverjüngend auf die Längsschlitze 6 auswirken und diese im Wesentlichen mittig über deren Längserstreckung teilen. Im Verbindungsbereich 7 ist das Innenteil 3 umfangsseitig fest mit der Außenhülse 2 verbunden und stirnseitig mit dem Saugrohr 10 gekoppelt, das wiederum über einen Saugschlauch mit einem Unterdrucksauger in Verbindung steht. Zur ermüdungsfreien Handhabung ist das Saugrohr 10 an beiden Enden unter Einschluss stumpfer Winkel leicht gebogen und weist ein Griffstück 11 auf.

Beim Absaugen von Blut strömt dasselbe durch die zwischen den Rippen 8 und den Längsschlitzen 6 vorhandenen Freiräume 9, die so bemessen sind, dass die roten Blutkörperchen nicht geschert werden und das Blut beim Absaugen darüber hinaus nicht in Turbulenz gerät, in den Saugkopf 1. Im weiteren Verlauf wird das Blut außenseitig über das Innenteil 3 zur Spitze geleitet und gelangt stirnseitig in eine Zentralbohrung 12 des Innenteils 3 und wird über das Saugrohr 10 abtransportiert.

Um einen kostengünstigen Einwegartikel bereitzustellen, sind die Außenhülse 2 und das Innenteil 3 des Saugkopfes 1 sowie das Saugrohr 10 mit dem Griffstück 11 aus Kunststoff gefertigt, wobei insbesondere die Außenhülse 2 aus einem transparenten Kunststoff gefertigt ist, damit ein Benutzer des Saugkopfes 1 das Absaugen des Blutes optisch überwachen kann.

## Patentansprüche

1. Saugkopf zur Absaugung des während eines chirurgischen Eingriffs sich im Eingriffsbereich ansammelnden Blutes, insbesondere im Herz- und Thoraxbereich bei Herzoperationen, mit mehreren umfangsseitigen Längsschlitzen (6), wobei eine Außenhülse (2) des Saugkopfes (1) Längsschlitze (6) aufweist, in die korrespondierende Rippen (8) eines in die Außenhülse (2) eingesetzten Innenteils (3) querschnittsverjüngend eingreifen, **dadurch gekennzeichnet, dass** das Innenteil (3) im Querschnitt sternförmig unter Ausbildung der Rippen (8) ausgebildet ist, zwischen den Rippen (8) und den Längsschlitzen (6) Freiräume (9) gebildet sind, die eine Strömungsverbindung in den Saugkopf (1) beim Absaugen schaffen, das rohrförmige Innenteil (3) stirnseitig mit einem Saugrohr (10) verbunden und in diesem endseitigen Verbindungsbereich (7) fest mit der Außenhülse (2) gekoppelt ist und außerhalb des Verbindungsbereichs (7) das Innenteil (3) zu der AuBenhülse (2) bereichsweise beabstandet ist, um Strömungskanäle auszubilden.

2. Saugkopf nach Anspruch 1, **dadurch gekennzeichnet, dass** der frei durchströmbare Querschnitt zwischen den Längsschlitzen (6) und den Rippen (8) derart bemessen ist, dass rote Blutkörperchen beim Durchströmen nicht geschert werden.

3. Saugkopf nach Anspruch 1 oder 2, **dadurch gekennzeichnet, dass** das Innenteil (3) kraftschlüssig mit dem Saugrohr (10) in Verbindung steht, insbesondere über einen Luer-Lock-Anschluss.

4. Saugkopf nach Anspruch 1 oder 2, **dadurch gekennzeichnet, dass** sich die Rippen (8) und die Längsschlitze (6), ausgehend von dem Verbindungsbereich (7), bis zu einer Spitze der Außenhülse (2) erstrecken.

5. Saugkopf nach Anspruch 4, **dadurch gekennzeichnet, dass** die Spitze der Außenhülse (2) einen Kugelradius (4) aufweist.

6. Saugkopf nach Anspruch 1, **dadurch gekennzeichnet, dass** die Außenhülse (2) an ihrer freien Stirnseite mit einer Saugöffnung (5) versehen ist, die einen Querschnitt aufweist, der derart bemessen ist, dass Gase und Flüssigkeiten mit einer geringeren Viskosität als Blut eingesaugt werden.

7. Saugkopf nach Anspruch 1, **dadurch gekennzeichnet, dass** die Außenhülse (2) und/oder das Innenteil (3) aus Kunststoff gefertigt sind.

8. Saugkopf nach Anspruch 7, **dadurch gekennzeichnet, dass** die Außenhülse (2) aus einem transparenten Kunststoff gefertigt ist und sich zu ihrem freien Ende hin konisch verjüngt.

9. Saugkopf nach Anspruch 7, **dadurch gekennzeichnet, dass** die Außenhülse (2) gemeinsam mit dem Innenteil (3) in einem Zwei-Komponenten-Spritzgußverfahren gefertigt ist.

10. Saugkopf nach Anspruch 1, **dadurch gekennzeichnet, dass** das Saugrohr (10) aus einem Kunststoff gefertigt ist.

11. Saugkopf nach Anspruch 1 oder 10, **dadurch gekennzeichnet, dass** das Saugrohr (10) ein Griffstück (11), insbesondere aus Kunststoff, aufweist.

12. Saugkopf nach einem der Ansprüche 1 bis 11, **dadurch gekennzeichnet, dass** das Saugrohr (10) einen Luer-Konus, insbesondere mit Überwurfmutter für einen Luer-Lock-Anschluss, zur Kopplung mit einem Saugschlauch einer Saugvorrichtung aufweist.

## Claims

1. A suction head for the aspiration of the blood accumulating in the intervention area during a surgical intervention, in particular in the cardiac and thoracic region during cardiac operations, with a plurality of circumferential longitudinal slots (6), wherein an outer sleeve (2) of the suction head (1) has longitudinal slots (6), into which corresponding ribs (8) of an inner part (3), inserted into the outer sleeve (2), engage, narrowing the cross-section, **characterized in that** the inner part (3) is constructed in a star-shaped manner in cross-section with the formation of the ribs (8), between the ribs (8) and the longitudinal slots (6) free spaces (9) are formed, which create a flow connection into the suction head (1) during aspirating, the tubular inner part (3) is connected on the face side with a suction tube (10) and in this end-side connecting region (7) is securely coupled with the outer sleeve (2) and outside the connecting region (7) the inner part (3) is spaced apart in certain areas with respect to the outer sleeve (2), in order to form flow channels.

2. The suction head according to Claim 1, **characterized in that** the cross-section between the longitudinal slots (6) and the ribs (8), which is able to be flowed through freely, is dimensioned such that red blood corpuscles are not sheared whilst flowing through.

3. The suction head according to Claim 1 or 2, **characterized in that** the inner part (3) is connected in a force-fitting manner with the suction tube (10), in particular via a luer-lock connection.

4. The suction head according to Claim 1 or 2, **characterized in that** the ribs (8) and the longitudinal slots (6), starting from the connection region (7), extend up to a tip of the outer sleeve (2).

5. The suction head according to Claim 4, **characterized in that** the tip of the outer sleeve (2) has a sphere radius (4).

6. The suction head according to Claim 1, **characterized in that** the outer sleeve (2) is provided on its free face side with a suction opening (5), which has a cross-section which is dimensioned such that gases and fluids with a lower viscosity than blood are sucked in.

7. The suction head according to Claim 1, **characterized in that** the outer sleeve (2) and/or the inner part (3) are made from plastic.

8. The suction head according to Claim 7, **characterized in that** the outer sleeve (2) is made from a transparent plastic and narrows conically towards its free end.

9. The suction head according to Claim 7, **characterized in that** the outer sleeve (2) together with the inner part (3) is produced in a two-component injection moulding process.

10. The suction head according to Claim 1, **characterized in that** the suction tube (10) is made from a plastic.

11. The suction head according to Claim 1 or 10, **characterized in that** the suction tube (10) has a grip piece (11), in particular of plastic.

12. The suction head according to one of Claims 1 to 11, **characterized in that** the suction tube (10) has a luer cone, in particular with a coupling nut for a luer-lock connection, for coupling with a suction hose of a suction device.

## Revendications

1. Tête d'aspiration destinée, au cours d'une intervention chirurgicale, à aspirer le sang s'accumulant dans la zone d'intervention, en particulier au niveau du coeur et du thorax dans les opérations cardiaques, comprenant plusieurs fentes longitudinales (6) sur le pourtour, sachant qu'un manchon externe (2) de la tête d'aspiration (1) présente des fentes longitudinales (6) dans lesquelles des nervures correspondantes (8) d'une pièce interne (3) insérée dans le manchon externe (2) se mettent en prise avec un rétrécissement en section transversale, **caractérisée en ce que** la pièce interne (3) a une section transversale en étoile par la formation des nervures (8), des espaces libres (9) sont formés entre les nervures (8) et les fentes longitudinales (6), qui créent une liaison d'écoulement dans la tête d'aspiration (1) lors de l'aspiration, la pièce interne tubulaire (3) est reliée par l'avant à un tube d'aspiration (10) et est couplée fixement au manchon externe (2) dans cette partie de liaison d'extrémité (7) et, hors de la partie de liaison (7), la pièce interne (3) est éloignée du manchon externe (2) par endroits, pour créer des canaux d'écoulement.

2. Tête d'aspiration selon la revendication 1, **caractérisée en ce que** la section pouvant être traversée librement entre les fentes longitudinales (6) et les nervures (8) a des dimensions telles que les globules rouges ne sont pas cisaillés lors de l'écoulement.

3. Tête d'aspiration selon la revendication 1 ou 2, **caractérisée en ce que** la pièce interne (3) est en liaison par adhérence de forces avec le tube d'aspiration (10), en particulier par un raccord luer-lock.

4. Tête d'aspiration selon la revendication 1 ou 2, **caractérisée en ce que** les nervures (8) et les fentes longitudinales (6), partant de la partie de liaison (7), s'étendent jusqu'à une pointe du manchon externe (2).

5. Tête d'aspiration selon la revendication 4, **caractérisée en ce que** la pointe du manchon externe (2) présente un rayon sphérique (4).

6. Tête d'aspiration selon la revendication 1, **caractérisée en ce que** le manchon externe (2) est doté d'une ouverture d'aspiration (5) sur sa face avant libre, qui présente une section transversale dont les dimensions permettent à des gaz et des liquides d'être aspirés avec une viscosité inférieure au sang.

7. Tête d'aspiration selon la revendication 1, **caractérisée en ce que** le manchon externe (2) et/ou la pièce interne (3) sont fabriqués en plastique.

8. Tête d'aspiration selon la revendication 7, **caractérisée en ce que** le manchon externe (2) est fabriqué dans un plastique transparent et présente un rétrécissement conique en direction de son extrémité libre.

9. Tête d'aspiration selon la revendication 7, **caractérisée en ce que** le manchon externe (2) conjointement avec la pièce interne (3), est fabriqué dans un procédé de moulage par injection bi-composants.

10. Tête d'aspiration selon la revendication 1, **caractérisée en ce que** le tube d'aspiration (10) est fabriqué en plastique.

11. Tête d'aspiration selon la revendication 1 ou 10, **caractérisée en ce que** le tube d'aspiration (10) présente une pièce de préhension (11), en particulier en plastique.

12. Tête d'aspiration selon l'une des revendications 1 à 11, **caractérisée en ce que** le tube d'aspiration (10) présente un cône luer, en particulier avec écrou-raccord pour un raccord luer lock, pour être couplé à un flexible d'aspiration d'un dispositif d'aspiration.
